# EUROPÄISCHE PATENTSCHRIFT

(11) **EP 1 412 068 B1**
(45) Veröffentlichungstag und Bekanntmachung des Hinweises auf die Patenterteilung: **15.02.2006**
(21) Anmeldenummer: 02764723.9
(22) Anmeldetag: 18.07.2002
(51) Int. Cl.: B01F 17/00, A61K 8/06, A61K 9/107

(54) **EMULGATOR-GEMISCH**
EMULSIFIER MIXTURE
MELANGE EMULSIFIANT

(30) Priorität: 27.07.2001 DE 10136483
(43) Veröffentlichungstag der Anmeldung: 28.04.2004
(73) Patentinhaber: Cognis IP Management GmbH, 40589 Düsseldorf (DE)
(72) Erfinder: BRÜNING, Stefan, Philadelphia, PA 19118 (DE); ANSMANN, Achim, 40699 Erkrath (DE); GONDEK, Helga, 40589 Düsselford (DE)
(86) Internationale Anmeldenummer: PCT/EP2002/008012
(87) Internationale Veröffentlichungsnummer: WO 2003/011421

(56) Entgegenhaltungen:
- EP-A- 0 771 558
- WO-A-00/04230
- WO-A-01/52806
- WO-A-94/14877
- WO-A-94/21593
- WO-A-94/22414
- WO-A-99/66898

## Beschreibung

### Gebiet der Erfindung

Die Erfindung betrifft ein neues Emulgator-Compound mit definierten Gehalten an Fettalkholen, ethoxylierten Fettalkoholen und ausgewählten Öl- und Wachskomponenten sowie die Verwendung der Emulgator-Compounds zur Herstellung von kosmetischen und/oder pharmazeutischen Zubereitungen.

### Stand der Technik

Im Bereich kosmetischer Emulsionen für die Haut- und Haarpflege werden vom Verbraucher eine Vielzahl von Anforderungen gestellt: abgesehen von den reinigenden und pflegenden Effekten, die den Anwendungszweck bestimmen, wird Wert auf so unterschiedliche Parameter wie höchstmögliche dermatologische Verträglichkeit, elegantes Erscheinungsbild, optimaler sensorischer Eindruck und Lagerstabilität gelegt. Einige dieser Merkmale, wie z.B. die Hautverträglichkeit lassen sich vom Kosmetikchemiker weitgehend objektiv bestimmen, kommt es jedoch zum sensorischen Eindruck so ist die Beurteilung durch Probanden letztlich immer subjektiv.

Bezüglich der Emulsionstechnologie ist es besonders wichtig, Emulgatoren und Emulgator-Kombinationen zur Verfügung zu stellen, die feinteilige und lagerstabile Emulsionen liefem. Demzufolge besteht ein kontinuierliches Interesse an der Entwicklung neuer Emulgator-Kombinationen, die es erlauben, besonders stabile, feinteilige Emulsionen zu formulieren. In diesem Zusammenhang sei auf die Aufsätze von A. Ansmann **[Seifen-Öte-Fette-Wachse, 117, 518 (1991)]**, C. Cabeta [**SÖFW-Journal, 120, 162 (1994)**], P.Hameyer [SÖFW-Journal, 121, 216, (1995)] und insbesondere A. Wadle [**Parf.Kosm. 77, 250 (1996)**] verwiesen.

Obschon dem Fachmann Maßnahmen bekannt sind, mit deren Hilfe er grundsätzlich zu feinteiligen und lagerstabilen Emulsionen gelangen kann, sind die Emulsionen des Stands der Technik - bedingt durch die Auswahl der eingesetzten Emulgatoren - nach wie vor nicht völlig zufriedenstellend.
Emulsionen werden üblicherweise durch eine Kombination von Emulgatoren stabilisiert. Häufig werden hierzu autoemulgierbare Mischungen auf Basis von Fettalkoholen eingesetzt, wie sie beispielsweise aus der **EP 0 553 241** (SEPPIC) oder der WO 97/18033 bekannt sind (Fettalkohol/Alkylpolyglycosid-Mischungen). Die im Handel unter den Bezeichnungen Cetomacrogol® Emulsifying Wax (British Pharmacopeia), Cire de Lanol® CTO (Seppic S.A.), Sinnowax® AO (Cognis France, S.A.), Promulgen® D (Amerchol) erhältliche Emulgator-Gemische seien nur beispielhaft für die Kombination Fettalkohol/Fettalkholethoxylat genannt. Emulgator-Gemische auf Basis Fettalkohol/Alkylpolyglucosid/Partialglycerid sind aus der Patentanmeldung **WO 92/07543** bekannt.

Die im Handel derzeit erhältlichen Emulgator-Gemische sind zur Herstellung dünnflüssiger O/W-Emulsionen, die auf dem Kosmetik-Markt derzeit einen wachsenden Absatz finden, oft ungeeignet. Neben geringer Emulsionsstabilität treten häufig Viskositätsänderungen bei Lagerung ein, insbesondere bei erhöhter Temperatur. Gleichzeitig zeigt sich häufig eine Abhängigkeit der Viskosität von der Schergeschwindigkeit beim großtechnischen Herstellungsprozeß, so daß man aus unterschiedlichen Produktionsanlagen Produkte mit unterschiedlichen Viskositäten erhält. Für die Herstellung kosmetischer Produkte und den Verbraucher sind derartige Schwankungen nicht akzeptabel.

Die Aufgabe der Erfindung hat somit darin bestanden, Emulgator-Gemische (Compounds) zur Herstellung kosmetischer Produkte - insbesondere von dünnflüssigen, niedrigviskosen O/W-Emulsionen - zur Verfügung zu stellen, die es erlauben, stabile Emulsionen zu erhalten, deren Viskosität unabhängig von der Schergeschwindigkeit beim Herstellungsprozeß ist, und sich auch bei Lagerung unter Temperaturbelastung (-5 bis 50°C) nicht ändert. Darüberhinaus sollten die Emulgator Gemische hohe Pflege-Effekte und verbesserte sensorische Eigenschaften aufweisen, welche die Herstellung sensorisch leichter Produkte erlauben. Ein weiterer Teilaspekt der Aufgabe bestand darin, Emulgator-Mischungen zur Verfügung zu stellen, welche es erlauben, die im Fachjargon als "Weißeln" bezeichnete Mikroschaumbildung auf der Haut zu reduzieren. In einem weiteren Teilaspekt der Aufgabe sollten Emulgator-Mischungen entwickelt werden, die es ermöglichen, stabile Emulsionen mit einem hohen Salzanteil herzustellen.

### Beschreibung der Erfindung

Es wurde gefunden, daß Emulgator-Gemische mit einem definierten Gehalt an Fettalkoholen, Fettalkoholethoxylaten und speziellen Öl- oder Wachskörpem nicht nur ausgezeichnete sensorische und pflegende Eigenschaften aufweisen, sondem bezüglich emulsionstechnischer Eigenschaften Vorteile bieten.
Gegenstand der Erfindung sind Emulgator-Gemisch enthaltend
(a) 0,1 - 60 Gew.-% eines Fettalkohols oder Fettalkohol-Gemisches
(b) 30 - 97 Gew.-% eines ethoxylierten Fettalkohols oder Gemisches ethoxylierter Fettalkohole
(c) 0,1- 20 Gew.-% eines Dialk(en)ylethers, eines Dialk(en)ylcarbonats oder eines beliebigen Gemisches dieser Komponenten
(d) weniger als 10 Gew.-% Wasser.

Die Komponenten (a), (b) und (c) der erfindungsgemäßen Emulgator-Zusammensetzungen können sich auch zu 100 % ergänzen, d.h. die Emulgatorzusammensetzung bestünde im wesentlichen, bis auf rohstoffbedingte Nebenprodukte und Restwassermengen aus diesen Komponenten, - wobei allerdings rohstoffbedingte Nebenprodukte oder Restwassermengen enthalten sein können. Das erfindungsgemäße Emulgatorgemisch kann aber auch noch weitere Komponenten enthalten. Die Wassermenge im Emulgatorgemisch beträgt weniger als 10 Gew.-%, vorzugsweise weniger als 5 Gew.-%.

Mit den erfindungsgemäßen Emulgator-Mischungen lassen sich sehr stabile Emulsionen herstellen, insbesondere dünnflüssige, niedrigviskose O/W-Emulsionen. Die resultierenden Emulsionen sind besonders lagerstabil und zeigen auch bei Temperaturbelastung (Lagertestbedingungen: 84 Tage bei - 5 - 50 °C) keine signifikanten Viskositätsschwankungen. Mit den erfindungsgemäßen "Compounds" lassen sich Emulsionen formulieren, deren Viskosität unabhängig von der Schergeschwindigkeit beim Herstellungsprozeß ist. Die erfindungsgemäße Kombination trägt desweiteren zu einer Reduktion der Mikroschaumbildung bei, die häufig in Emulsionen mit Wachskomponenten auftritt. Im speziellen lassen sich bei Verwendung der erfindungsgemäßen Emulgatormischung sehr leicht stabile Emulsionen mit höheren Salzkonzentrationen herstellen, insbesondere dünnflüssige Antitranspirant-Roll-on-Formulierungen mit Aluminiumchlorohydraten und ähnlichen Salzen.

Eine bevorzugte Ausführungsform des Emulgatorgemisches enthält eine Kombination aus (a) 2 - 60 Gew.% eines Fettalkohols oder Fettalkohol-Gemisches, (b) 30 - 90 Gew.-% eines ethoxylierten Fettalkohols oder Gemisches ethoxylierter Fettalkohole und (c) 0,2 - 15 Gew.-% eines Dialk(en)ylethers, eines Dialk(en)ylcarbonats oder eines beliebigen Gemisches dieser Komponenten. Eine besonders bevorzugte Ausführungsform enthält eine Kombination aus (a) 10 - 55 Gew.-% eines Fettalkohols oder Fettalkohol-Gemisches, (b) 30 bis 85 Gew.-% eines ethoxylierten Fettalkohols oder Gemisches ethoxylierter Fettalkohole und (c) 0,5 - 15 Gew.-% eines Dialk(en)ylethers, eines Dialk(en)ylcarbonats oder eines beliebigen Gemisches dieser Komponenten.

Ein weiterer Gegenstand der Erfindung ist die Verwendung der erfindungsgemäßen Emulgator-Gemische zur Herstellung kosmetischer und pharmazeutischer Mittel. Vorzugsweise werden die Emulsions-Gemische zur Herstellung von O/W-Emulsionen eingesetzt. Besonders bevorzugt werden sie zur Stabilisierung von dünnflüssigen O/W-Emulsionen mit Viskositäten von 100 - 20000 mPa·s (Brookfield, RVF, Spindel 5,10 upm, 23°C), insbesondere zur Herstellung von Antitranspirant-Roll-on-Formulierungen eingesetzt.

### Fettalkohole (Emulgatorkomponente a)

Unter Fettalkoholen sind primäre aliphatische, verzweigte oder unverzweigte, gesättigte oder ungesättigte, ggf. hydroxysubstituierte Alkohole mit einem Kohlenwasserstoffrest von 6 bis 54 Kohlenstoffatomen zu verstehen.

Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Isocetylalkohol, Palmoleyl-alkohol, Stearylalkohol, Isostearylalkohol, Oleylalkohol, Elaidylalkohol, Petroselinylalkohol, Linofylalkohol, Linolenylalkohol, Elaeostearylalkohol, Ricinolalkohol, Hydroxystearyl-alkohol, Dihydroxystearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestem auf Basis von Fetten und Ölen oder Aldehyden aus der Roelen'schen Oxosynthese sowie als Monomerfraktion bei der Dimerisierung von ungesättigten Fettalkoholen anfallen. Weitere Beispiele sind die Guerbetalkohole auf Basis von Fettalkoholen mit 6 bis 18 Kohlenstoffen sowie technische Dimerdiole und Trimertriole mit 18 bis 36 bzw. 18 bis 54 Kohlenstoffatomen, die aus der Oligomerisierung und nachfolgenden Hydrierung ungesättigter Fettsäuren stammen.

Emulgator-Mischungen, bei denen die Fettalkohol-Komponente (a) gewählt ist aus der Gruppe der C₁₂-C₂₄-Fettalkohole sind erfindungsgemäß bevorzugt. Besonders bevorzugt ist es, technische Fettalkohole mit 16 bis 18 Kohlenstoffatomen wie beispielsweise Cetylstearylalkohol, Isostearylalkohol sowie Guerbetalkohole entsprechender Kettenlänge einzusetzen. Besonders bevorzugt ist der Einsatz von C₁₆-, C₁₈- sowie C₁₆/C₁₈-Fettalkoholen in einer Menge von 0,1- 60 Gew.-% und insbesondere 10 - 55 Gew.-% bezogen auf die Gesamtzusammensetzung des Emulgator-Gemisches.

### Fettalkoholethoxylate (Komponente b)

Fettalkoholethoxylate, die als Komponente (b) in Frage kommen, stellen Anlagerungsprodukte von Ethylenoxid an primäre Alkohole dar. Die Ethoxylate werden nach den hinreichend bekannten Verfahren des Standes der Technik hergestellt und sind grundsätzlich Mischungen. Herstellungsbedingt können sie eine konventionell breite oder auch eingeengte Homologenverteilung aufweisen. Der Ethoxylierungsgrad (E0: Anzahl der angelagerten Ethylenoxid-Einheiten) stellt dabei eine Gauss-Verteilung dar, wobei das Maximum der Gauss-Kurve hier als mittlerer Ethoxylierungsgrad n bezeichnet wird.

Erfindungsgemäß bevorzugt ist eine Kombination von Anlagerungsprodukten mit niedrigem (n = 1 - 5 EO) und höherem mittleren Ethoxylierungsgrad (n = 6 - 35 EO). Die EO-Angaben beziehen sich jeweils auf das Maximum der Gauss-Verteilung, also den mittleren Ethoxylierungsgrad. Vorzugsweise enthält die Komponente (b) des erfindungsgemäßen Emulgator-Gemisches eine Kombination aus (b1) einem C₁₂-C₂₄-Fettalkohol mit 1 - 5 EO und (b2) einem C₁₂-C₂₄-Fettalkohol mit 6 - 35 EO. Erfindungsgemäß besonders bevorzugt ist es, eine Dreier-Kombination einzusetzen, bei der die Komponente (b) ein Gemisch aus (b1) C₁₂ - C₂₄-Fettalkoholen mit 1 - 5 EO, (b2) C₁₂ - C₂₄₋Fettalkoholen mit 6 -16 EO und (b3) C₁₂ - C₂₄-Fettalkoholen mit 18 - 35 EO enthält. Ganz besonders bevorzugt ist die Kombination (b1) C₁₈-Fettalkohol mit 1-2 EO, (b2) C₁₆/C₁₈-Fettalkohol mit 10-12 EO und (b3) C₁₆/C₁₈-Fettalkohol mit 18-22 EO. Die Dreier-Kombination ist emulsionstechnisch vorteilhaft, so daß besonders stabile Emulsionen gebildet werden, und führt auch zu sensorisch besseren Produkten. Eine besonders bevorzugte Kombination der Komponente (b) ist eine Gemisch aus Eumulgin® S, Eumlgin® B1 und Eumulgin® B2, die von der Cognis Deutschland GmbH vertrieben werden. Eumulgin® S ist ein Gemisch aus Stearylalkohol und Steareth-2.

Die Herstellung derartiger nichtionischer Tenside entspricht dem oben ausgeführten. Typische Beispiele sind Anlagerungsprodukte von Ethylenoxid an Capronalkohol, Caprylalkohol, 2-Ethylhexylalkohol, Caprtnalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind die Addukte von Ethylenoxid an Cetearylalkohol, Stearlyalkohol, Isostearylalkohol oder Behenylalkohol.

### Dialk(en)ylether und Dialk(en)ylcarbonate (Komponente C)

Die gezielte Auswahl der Komponente (c) verleiht der Emulgator-Mischung, auch bei Zusatz einer relativ kleinen Menge, sensorisch bessere Eigenschaften. Die resultierenden Emulsionen, die mit der erfindungsgemäßen Mischung formuliert werden, sind stabiler und vermitteln ein leichteres, und weniger fettendes Hautgefühl. Die Komponente (c) ist in einer Menge von 0,1 - 20 Gew.-%, vorzugsweise 0,2 - 15 Gew.-%, 0,5 - 15 und insbesondere 0,5 - 5 Gew.-% bezogen auf die Gesamtzusammensetzung des Emulgator-Gemisches enthalten.

Die Dialk(en)ylether können symmetrisch oder unsymmetrisch, verzweigt oder unverzweigt, gesättigt oder ungesättigt sein. Erfindungsgemäß bevorzugt geeignet sind gesättigte C₆-C₃₂-Dialkylether, wie beispielsweise Di-n-octylether, Di-(2-ethylhexyl)-ether, Laurylmethylether oder Octylbutylether, sowie Didodecylether. Besonderes bevorzugt geeignet sind C₁₆-C₃₂-Dialkylether, da sie zusätzlich konsistenzgebende Eigenschaften haben. Besonders bevorzugt sind C₁₆-C₂₄-Dialkylether, und insbesondere bevorzugt geeignet ist Distearylether und Dibehenylether.

Die Verbindungen lassen sich aus Fettalkoholen in Gegenwart saurer Katalysatoren nach allgemein bekannten Verfahren des Standes der Technik herstellen, z. B. **DE 19511668 A1** und **DE 198 31705 A1** sowie **DE 199 43 585**. Typische Beispiele für derartige Ether sind Produkte, die durch Veretherung von Capronalkohol, Caprylalkohol, 2-Ethylhexylalkohol, Caprinalkohol, Laurylalkohol, Myristylalkohol, Cetylalkohol, Palmoleylalkohol, Stearylalkohol, Isostearylalkohol, Elaidylalkohol, Petroselinylalkohol, Linolylalkohol, Linolenylalkohol, Oleylalkohol, Rizinolalkohol, Elaeostearylalkohol, Arachidylalkohol, Gadoleylalkohol, Behenylalkohol, Erucylalkohol und Brassidylalkohol, Guerbetalkoholen, sowie deren technische Mischungen, die z.B. bei der Hochdruckhydrierung von technischen Methylestem auf Basis von Fetten und Ölen anfallen, gewonnenen werden.

Die **Dialk(en)ylcarbonate** können symmetrisch oder unsymmetrisch, verzweigt oder unverzweigt, gesättigt oder ungesättigt sein. Unter den Dialk(en)ylcarbonaten sind lineare oder verzweigte, gesättigte oder ungesättigte C₆-C₃₂-Dialkyl(en)carbonate erfindungsgemäß bevorzugt, wie z. B. Dihexyl-, Dioctyl-, Di-(2-ethylhexyl)- oder Dioleylcarbonat. Besonders bevorzugt sind sind wachsartige C₁₆-C₃₂-Dialkyl(en)carbonate, da sie konsistenzgebende Eigenschaften haben. Besonders bevorzugt sind C₁₆-C₂₄-Dialkyl(en)carbonate und unter diesen gesättigte, unverzweigte C₁₆-C₂₂-Dialkylcarbonate. Insbesondere bevorzugt geeignet ist Distearylcarbonat.

Die Verbindungen lassen sich durch Umesterung von Dimethyl- oder Diethylcarbonat mit den entsprechenden Hydroxyverbindungen nach Verfahren des Standes der Technik herstellen; eine Übersicht hierzu findet sich in **Chem.Rev. 96, 951 (1996).** Typische Beispiele für Dialkyl(en)carbonate sind Umesterungsprodukte von Dimethyl- und/oder Diethylcarbonat mit Capronalkohol, Caprylalkohol, 2-Ethylhexylalkohol, Caprinalkohol, Laurylalkohol, Myristylalkohol, Cetylalkohol, Palmoleylalkohol, Stearylalkohol, lsostearylalkohol, Elaidylalkohol, Petroselinylalkohol, Linolylalkohol, Linolenylalkohol, Oleylalkohol, Rizinolalkohol, Elaeostearylalkohol, Arachidylalkohol, Gadoleylalkohol, Behenylalkohol, Erucylalkohol und Brassidylalkohol, Guerbetalkoholen, sowie deren technische Mischungen, die z.B. bei der Hochdruckhydrierung von technischen Methylestem auf Basis von Fetten und Ölen anfallen.

Eine bevorzugte Ausführungsform der Emulgator-Mischung enthält folgende Komponenten:
(a) 0,1-60 Gew.-% eines C₁₂-C₂₄-Fettalkohols,
(b1) 10 - 65 Gew.-% eines C₁₂-C₂₄-Fettalkohols mit 1- 5 EO,
(b2) 10-30 Gew.-% eines C₁₂-C₂₄-Fettalkohols mit 6-16 EO,
(b3) 10 - 20 Gew.-% eines C₁₂-C₂₄-Fettalkohols mit 18 - 35 EO, und
(c) 0,1 bis 15 Gew.-% eines C₆-C₃₂-Dialk(en)ylethers, eines C₆-C₃₂₋Dialk(en)ylcarbonats oder eines beliebigen Gemisches dieser Komponenten

### Partialglyceride

In einer weiteren bevorzugten Ausführungsform enthält das erfindungsgemäße Emulgator-Gemisch zusätzlich ein Partialglycerid. Diese unterstützen den Aufbau lamellarer Phasen in der wäßrigen Phase der Emulsionen, was zu einer weiteren Verbesserung des Pflegeeffektes beim Auftrag auf die Haut führt. Das Pardalglycerid kann in einer Menge von 0,1-10 Gew.-%, vorzugsweise 0,1 - 5 Gew.-% und insbesondere 0,1 - 3 Gew.-% bezogen auf die Gesamtzusammensetzung des Emulgator-Gemisches enthalten.

Fettsäurepartialglyceriden, sind bekannte Stoffe, die nach einschlägigen Verfahren der präparativen organischen Chemie hergestellt werden können. Üblicherweise sind dies Mischungen von Mono- und Diglyceriden, die durch Umesterung der entsprechenden Triglyceride mit Glycerin oder durch gezielte Veresterung von Fettsäuren erhalten werden. Herstellungsbedingt können noch geringe Mengen an Triglyceriden enthalten sein. Die Abtrennung von nichtumgesetzten Ausgangsstoffen sowie die Anreicherung von Monoglyceriden in den Gemischen erfolgt üblicherweise über eine Molekulardestillation. Typische Beispiele sind technische Mono- und/oder Diglyceride, die sich von den folgenden Fettsäuren ableiten: Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, und bei der Oxidation von Aldehyden aus der Roelen'schen Oxosynthese anfallen. Besonders bevorzugt ist der Einsatz von Partialglyceriden auf Basis von Stearinsäure bzw. Talgfettsäure.

Unter den Fettsäuren sind erfindungsgemäß unverzweigte, ungesättigte C₈-C₂₄-Fettsäuren, und unter diesen insbesondere C₁₂-C₁₈-Fettsäuren bevorzugt. In einer bevorzugten Ausführungsform enthält das Emulgator-Gemisch ein C₁₂-C₁₈-Partialglycerid. Erfindungsgemäß besonders bevorzugt ist die Verwendung von Partialglyceriden, die aus der Gruppe der Glycerinmonoester ausgewählt sind oder von technischen Mischungen, die einen Glycerinmonoester-Anteil von 30 - 90 Gew.-% enthalten. Diese zeigen den optimalsten Aufbau lamellarer Phasen und damit den optimalsten Pflegeeffekt.

Eine weitere bevorzugte Ausführungsform der Emulgatormischung enthält:
(a) 0,1- 60 Gew.-% eines C₁₂ - C₂₄-Fettalkohols,
(b1) 10-65 Gew.-% eines C₁₂ - C₂₄-Fettalkohols mit 1- 5 EO,
(b2) 10 - 30 Gew.-% eines C₁₂ - C₂₄-Fettalkohols mit 6-16 EO,
(b3) 10 - 20 Gew.-% eines C₁₂ - C₂₄-Fettalkohols mit 18 - 35 EO,
(c) 0,1 bis 15 Gew.-% eines C₆ - C₃₂-Dialk(en)ylethers, eines C₆ - C₃₂₋Dialk(en)ylcarbonats oder eines beliebigen Gemisches dieser Komponenten
(e) 0,1 bis 10 Gew.-% eines C₁₂-C₁₈-Partialglycerids

### Kosmetische und pharmazeutische Mittel

Das erfindungsgemäße Emulgator-Compound wird vorteilhaft zur Herstellung kosmetischer und pharmazeutischer Zubereitungen zur Körperpflege und -reinigung eingesetzt, wie z. B. Lotionen, sprühbare Emulsionen, Sonnenschutzmittel, Shampoos, Duschbäder, Antitranspirantien, etc. Vorzugsweise handelt es sich hierbei um O/W-Emulsionen mit einem Wasseranteil von vorzugsweise mehr als 50 Gew.-% bezogen das Mittel. Diese Mittel enthalten üblicherweise eine Reihe weiterer, in der Kosmetik üblichen Stoffe, wie Fette, Öle, Esteröle, Wachse, Feuchthaltemittel und Hydrotrope, weitere Emulgatoren und Co-Emulgatoren, Tenside, Lösungsvermittler, entzündungshemmende Stoffe, Wirkstoffe, Verdickungsmittel, Pflanzenextrakte, etc. und Wasser. Exemplarisch seien hier, ohne darauf beschränkt zu sein, einige Stoffklassen aufgeführt.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀₋Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkyl(en)ether, Dilkyle(en)carbonate und/oder aliphatische bzw. naphthenische Kohlenwasserstoffe sowie Siliconöle, Dimethicone oder Cyclomethicone in Betracht. Die Menge der Ölkörper in den kosmetischen Mitteln beträgt üblicherweise 1 - 20 Gew.-%, vorzugsweise 1 - 15 Gew.-% bezogen auf die Gesamtzusammensetzung des Mittels.

### Tenside

Die mit Hilfe der erfindungsgemäßen Emulgatoren erhältlichen Emulsionen können je nach Applikationszweck als weitere Bestandteile zusätzliche anionische, nichtionische, kationische und/oder amphotere bzw. zwitterionische Tenside und Emulgatoren enthalten.

Typische Beispiele für **anionische Tenside**, die v.a. in Duschbädem und Shampoo-Formulierungen eingesetzt werden, sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischether-sulfate, Monoglycerid(ether)sulfate, Fettsäureamid-(ether)sulfate, Mono- und Dialkylsulfo-succinate, Mono- und Dialkylsulfosuccinamate, Sulfotriglyceride, Amidseifen, Ether-car-bonsäuren und deren Salze, Fettsäureisethionate, Fettsäuresarcosinate, Feftsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)-phosphate. Sofern die anionischen Tenside Polyglycoletherketten enthalten, können diese eine konventionelle, vorzugsweise jedoch eine eingeengte Homologenverteilung aufweisen.

Typische Beispiele für nichtionische Tenside/Emulgatoren sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether, Fettsäure-N-alkyl-glucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Typische Beispiele für **kationische Tenside** sind quartäre Ammoniumverbindungen und Esterquats, insbesondere quatemierte Fettsäuretrialkanolaminester-Salze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöl-additive", Thieme Verlag, Stuttgart, 1978, S. 123-217** verwiesen.

### Weitere Hilfs- und Zusatzstoffe

Die Emulsionen können als Hautpflegemittel, wie beispielsweise Tagescremes, Nachtcremes, Pflegecremes, Nährcreme, Bodylotions, Salben und dergleichen eingesetzt werden und als weitere Hilfs- und Zusatzstoffe Co-Emulgatoren, Kationpolymere, Silicone, Überfettungsmittel, Fette, Wachse, Stabilisatoren, biogene Wirkstoffe, Glycerin, Konservierungsmittel, Farb- und Duftstoffe enthalten.

Geeignete kationische Polymere sind beispielsweise kationische Cellulosederivate, kationische Stärke, Copolymere von Diallylammoniumsalze und Acrylamide, quatemierte Vinylpyrrolidon/Vinylimidazol-Polymere wie z.B. Luviquat® (BASF AG, Ludwigshafen/ FRG), Kondensationsprodukte von Polyglycolen und Aminen, quatemierte Proteinpoly-peptide wie beispielsweise Lauryldimonium hydroxypropyl hydrolyzed collagen (Lamequat®L, Grünau GmbH), Polyethylenimin, kationische Siliconpolymere wie z.B. Amido-methicone oder Dow Coming, Dow Coming Co./US, Copolymere der Adipinsäure und Dimethylaminohydroxypropyldiethylentrimamin (Cartaretine®, Sandoz/CH), Polyamino-polyamide wie z.B. beschrieben in der **FR-A 22 52 840** sowie deren vemetzte wasserlöslichen Polymere, kationische Chitinderivate wie beispielsweise quatemiertes Chitosan, gegebenen-falls mikrokristallin verteilt, kationischer Guar-Gum wie z.B. Jaguar® CBS, Jaguar® C-17, Jaguar® C-16 der Celanese/US sowie quatemierte Ammoniumsalz-Polymere wie z.B. Mira-pol® A-15, Mirapol® AD-1 und Mirapol® AZ-1 der Miranot/US.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor- und/oder alkylmodifizierte Siliconverbindungen. Als **Überfettungsmittel** können Substanzen wie beispielsweise polyethoxylierte Lanofinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Stabilisatoren** können Metallsalze von Fettsäuren wie z.B. Magnesium-, Aluminium und/oder Zinkstearat eingesetzt werden. Unter biogenen **Wirkstoffen** sind beispielsweise Pflanzenextrakte und Vitaminkomplexe zu verstehen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, Verlag Chemie, Weinheim, 1984, S.81-106** zusammengestellt sind.

### Antitranspirant-Formulierungen

Das erfindungsgemäße Emulgator-Gemisch läßt sich ganz besonders bevorzugt zur Formulierung von Deo- und Antitranspirant-Formulierungen einsetzen, die eine verbesserte Sensorik aufweisen. Insbesondere sind dünnflüssige, oder sogenannte Roll-on-Antitranspirant-Formulierungen aufgrund der hohen Salz-Konzentration häufig instabil, d.h. die Emulsion bricht oder verändert während der Lagerung besonders leicht die Viskosität. Je nach Schergeschwindigkeit im Herstellungsprozeß resultieren Antitranspirant-Produkte mit sehr unterschiedlicher Viskosität.

Mit dem erfindungsgemäßen Emulgator-Gemisch lassen sich dagegen sehr stabile und konstante Produkteigenschaften einstellen. Die Produkte weisen eine deutliche Reduktion der

Mikroschaumbildung ("Weißeln") auf. Ein bevorzugte Ausführungsform der kosmetischen Mittel betrifft daher Formulierungen, die Antitranspirant-Wirkstoffe enthält, insbesondere Roll-on-Formulierungen mit einer Viskosität von 1000 - 20000 mPa·s (Brookfield-RVT, Spindel 5,10 Upm, Temperatur 23°C).

Die Antitranspirant-Wirkstoffe sind in den erfindungsgemäßen Zusammensetzungen in einer Menge von 0,1 - 30 Gew.-%, vorzugsweise 5-25 Gew.-% und insbesondere 10-25 Gew.% enthalten (bezogen auf die Menge der Aktivsubstanz). Als Antitranspirant-Wirkstoffe kommen z. B. Aluminiumchlofiydrate, Aluminium-Zirkonium-Chlorohydrate sowie Zinksalze in Frage. Diese wirken wahrscheinlich über den partiellen Verschluß der Schweißdrüsen durch Eiweiß- und/oder Polysaccharidfällung. Neben den Chlorhydraten können auch Aluminiumhydroxylactate sowie saure Aluminium/Zirkoniumsalze eingesetzt werden. Unter der Marke Locron® der Clariant GmbH, befindet beispielsweise sich ein Aluminiumchlorhydrat im Handel, das der Formel [Al₂(OH)₅Cl]·2,5 H₂O entspricht, und dessen Einsatz besonders bevorzugt ist. Ebenso erfindungsgemäß bevorzugt ist der Einsatz von Aluminium-Zirkonium-Tetrachlorohydrex-Glycin-Kompfexen, die beispielsweise von Reheis unter der Bezeichnung Rezal® 36G vermarktet werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® C.A.T., Cognis Deutschland GmbH). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester. Antibakterielle Wirkstoffe, welche die Keimflora beeinflussen und schweißzersetzende Bakterien abtöten bzw. in ihrem Wachstum hemmen, können ebenfalls in den Zusammensetzungen enthalten sein. Beispiele hierfür sind Chitosan, Phenoxyethanol und Chlorhexidingluconat. Besonders wirkungsvoll hat sich auch 5-Chlor-2-(2,4-dichlorphenoxy)-phenol erwiesen, das unter der Bezeichnung Irgasan® von der Ciba-Geigy, Basel/CH vertrieben wird. Die Emulgator-Kombination ist aber auch vorteilhaft zur Formulierung von Antitranspirant-Cremes einsetzbar. Ein weiterer Gegenstand der Erfindung sind daher Antitranspirantcremes mit einer Viskosität von 20 - 800 Pa·s (Brookfield-RVT, Spindel 5, 10 Upm, Temperatur 23°C bei Viskositäten von 20 - 40 Pa.s und Brookfield-RVT, Spindel TE, 4 Upm, Temperatur 23°C, bei Viskositäten von 40 - 800 Pa·s).

### Sonnenschutz-Formulierungen

Auch Sonnenschutz-Zubereitungen sind mit der efindungsgemäßen Emulgator-Mischung besonders leicht formulierbar. Die UV-Lichtschutzfaktoren sind sehr leicht einarbeitbar, die Endformulierungen auch bei Lagerung unter Temperaturbelastung lange Zeit stabil. Ein weiterer Gegenstand der Erfindung sind daher kosmetische Zubereitungen, die UV-Lichtschutzfaktoren enthalten.

Die UV-Lichtschutzfaktoren sind in den erfindungsgemäßen Zusammensetzungen in einer Gesamtmenge von 1 - 30 Gew.-%, vorzugsweise 5 - 25 Gew.% und insbesondere 5 - 15 Gew.-% enthalten (bezogen auf die Menge der Aktivsubstanz).

Als **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UVB-Filter können öllöslich oder wasserlöslich sein. Als öllösliche Substanzen, die erfindungsgemäß bevorzugt sind, sind z. B. zu nennen:
- 3-Benzylidencampher bzw. 3-Benzylidennorcampher und dessen Derivate, z. B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester,
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon;
- Propan-1,3-dione, wie z.B. 1-(4-tert.Butylphenyl)-3-(4'methoxyphenyl)propan-1,3-dion;
- Ketotricyclo(5.2.1.0)decan-Derivate;

Als wasserlösliche UV-Filter-Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen verwendet Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex®T 2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt, z. B. mikronisiertes Zinkoxid.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z. B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Asc;orbylphosphat, Ascorbylacetat), Tocopherole und Derivate (z.B. Vitamin-E-acetat), Vitamin A und Derivate (Vtamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z. B. ZnO, ZnSO₄) Selen und dessen Derivate (z. B. Selen-Methionin), Stilbene und deren Derivate (z. B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Beispiele

Tabelle 1 enthält fünf Formulierungen (Compound A-E) für das efindungsgemäße Emulgator-Gemisch (Angaben in Gew.% bezogen auf die Gesamtzusammensetzung des Emulgator Compounds).

**Erfindungsgemäße Compounds A-E (Tabelle 1):**

| **Compound** | **A (w/w%)** | **B (w/w%)** | **C (w/w%)** | **D (w/w%)** | **E (w/w%)** |
|---|---|---|---|---|---|
| Eumulgin® S2 | 55 | 56 | 19 | 55 | 55,5 |
| Eumulgin® B1 | - | 25 | 25 | 25 | 25 |
| Eumulgin® B2 | 42 | 17 | 17 | 17 | 17 |
| Stearylalkohol | 1 | 1 | 37 | 1 | 1 |
| Dioctadecylether | 2 | 1 | 2 | - | 0,5 |
| Dioctadecylcarbonate | - | - | - | 2 | 0,5 |
| C₁₆/C₁₈-Fettsäure-Mono/Diglycerid (Cutina® MD) | - | - | - | - | 0,5 |

| | | | | | |
|---|---|---|---|---|---|
| Eumulgin® S2: Mischung aus 62,5 Gew.-% Steareth-2 und 37,5 Gew.-% Stearylalkohol | | | | | |
| Eumulgin® B1: Ceteareth-12 | | | | | |
| Eumulgin®B2: Ceteareth-20 | | | | | |

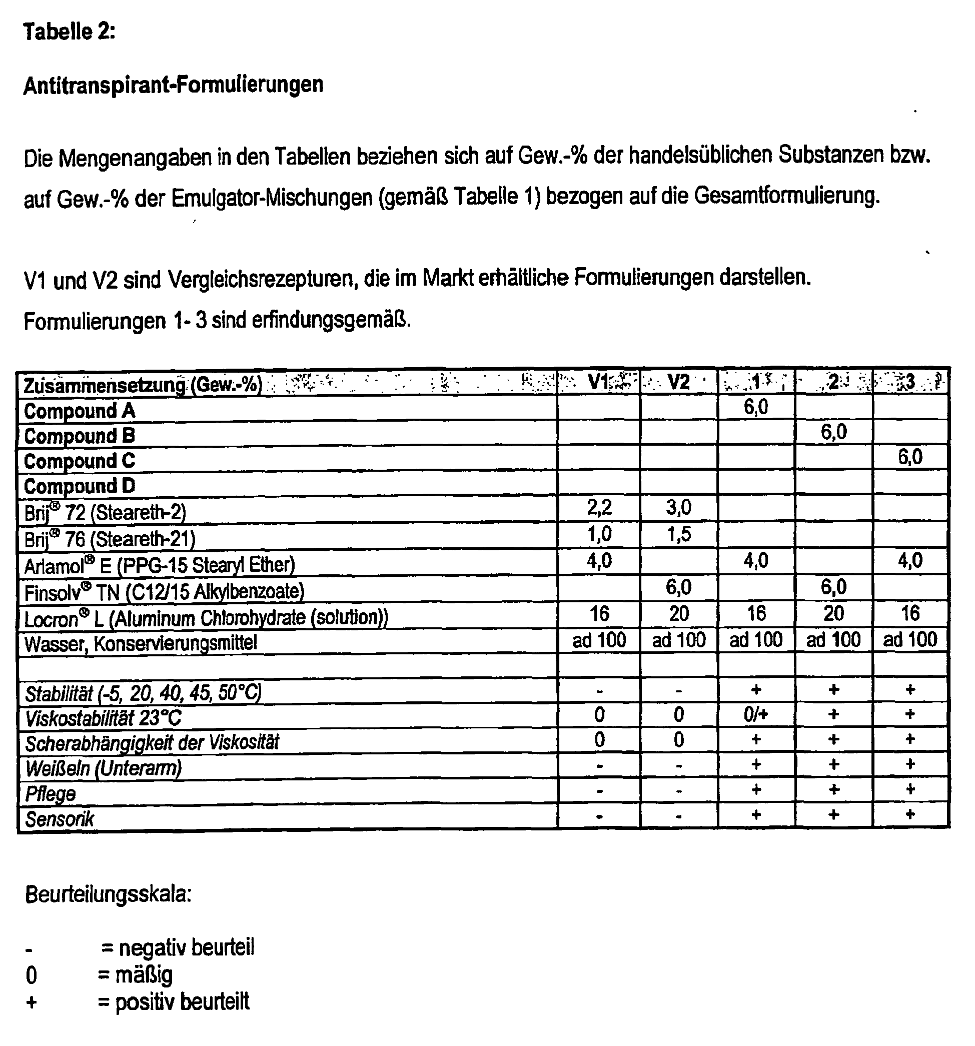

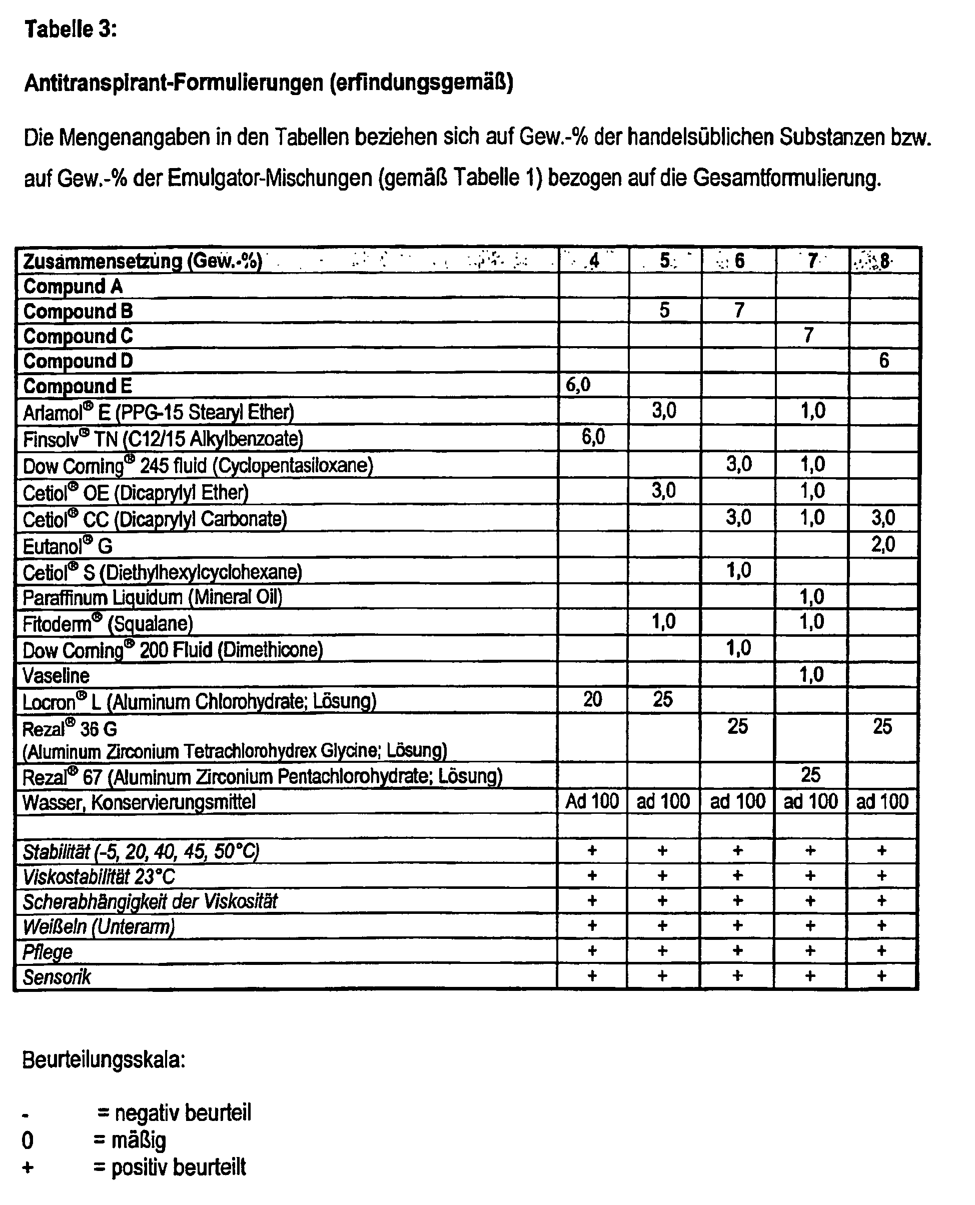

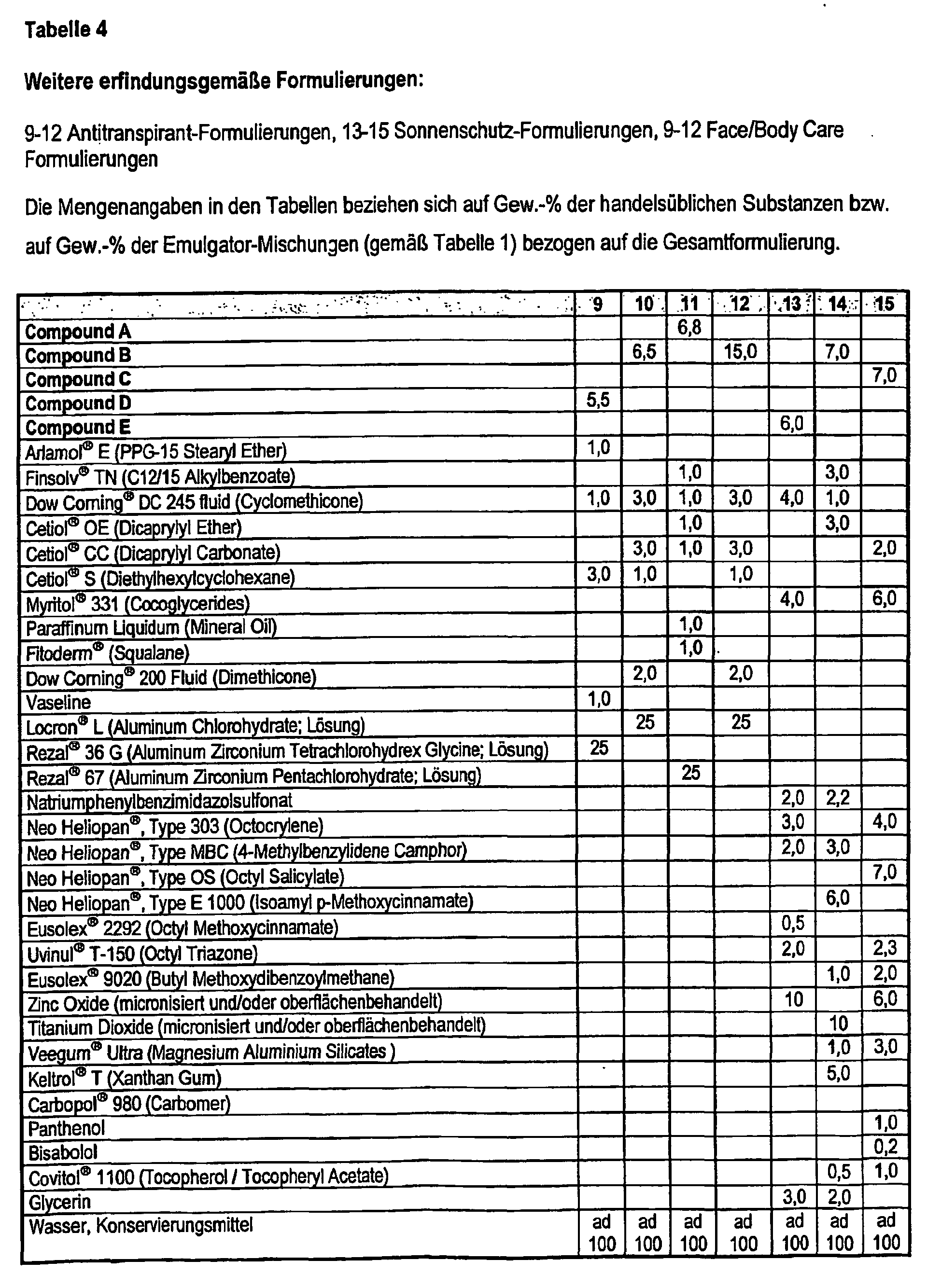

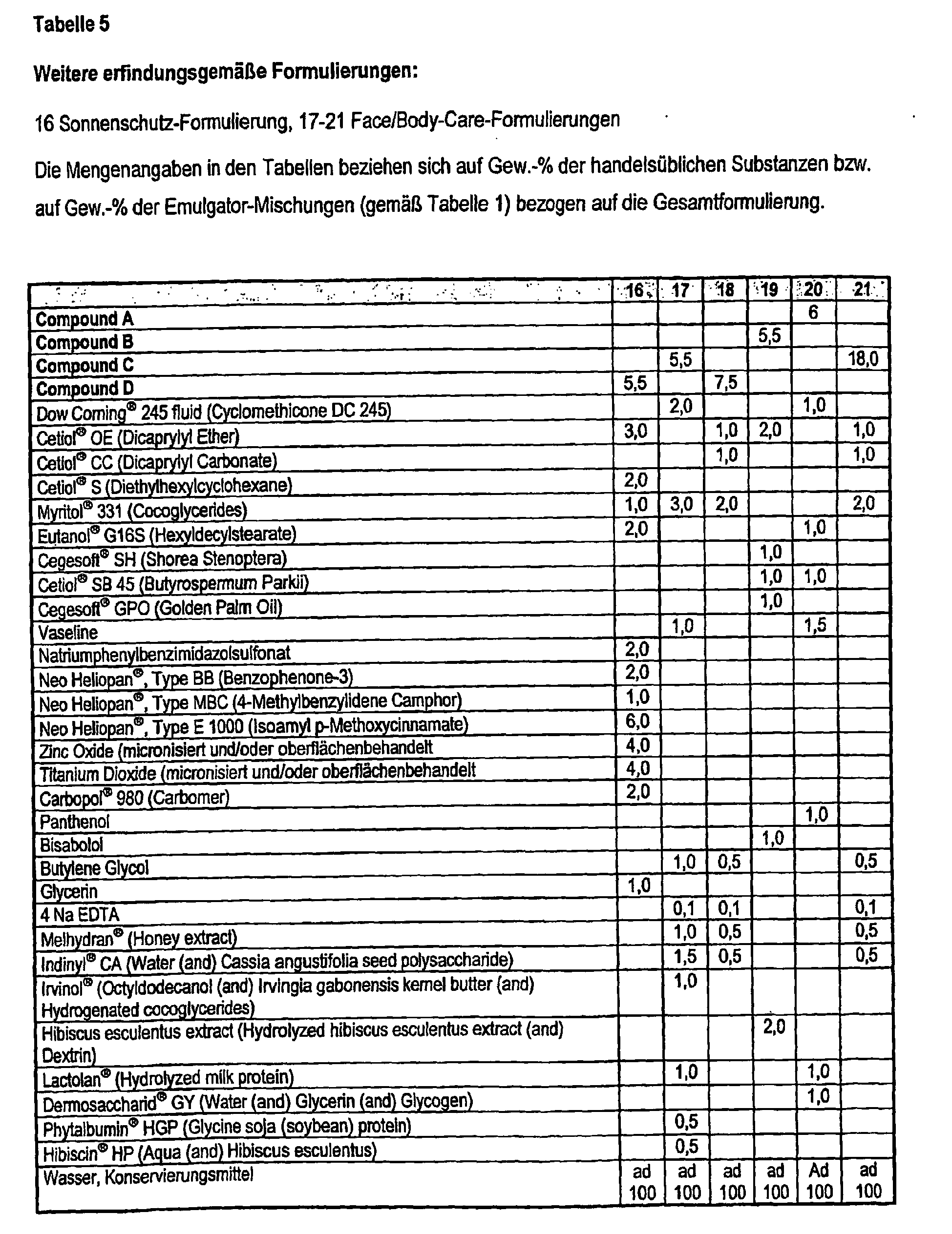

### Anhang

1. Ariamol® E
   INCI: PPG 15 Stearyl Ether
   Hersteller: Uniqema (Brenntag)
2. Brij® 72
   INCI: Steareth 2
   Hersteller: Uniqema (Brenntag)
3. Brij® 76
   INCI: Steareth 10
   Hersteller: Uniqema (ICI Surfactants)
4. Cegesoft® GPO
   INCI: Palm (Elaeis Guineensis) Oil
   Hersteller: Cognis Deutschland GmbH
5. Cetiol® CC
   Synonym: Dioctylcarbonat
   Hersteller. Cognis Deutschland GmbH (Henkel)
6. Cetiol® OE
   INCI: Dicaprylyl Ether
   Hersteller. Cognis Deutschland GmbH (Henkel)
7. Cetiol® S
   INCI: Dioctylcyclohexane (alt), Diethylhexylcyclohexane
   Hersteller: Cognis Deutschland GmbH (Henkel)
8. Cetiol® SB 45
   INCI: Shea Butter, Butyrospermum Parkii (Unne)
   Hersteller: Cognis Deutschland GmbH
9. Covitol® 1100
   INCI: Tocopheryl Acetate
   Hersteller. Cognis Corporation
10. Cutina® MD
   INCI: Glyceryl Stearate
   Hersteller: Cognis Deutschland GmbH
11. Dermosaccharid® GY
   INCI: Aqua (Water), Glycerin, Glycogen
   Hersteller. Laboratoires Serobiologiques (Cognis)
12. Dow Coming® 200 Fluid, 100 cS
   INCI: Dimethicone
   Hersteller: Dow Coming
13. Dow Coming® 245 Fluid
   INCI: Cyclopentasiloxane
   Hersteller: Dow Coming
14. Eumulgin® S2
   INCI: Steareth-2
   Hersteller: Cognis Deutschland GmbH
15. Eumulgin® B1
   INCI: Ceteareth-12
   Hersteller: Cognis Deutschland GmbH
16. Eumulgin® B2
   INCI: Ceteareth-20
   Hersteller: Cognis Deutschland GmbH
17. Eusolex® 9020
   INCI: Butyl Methoxydibenzoylmethane
   Hersteller: Merck
18. Eutanol® G16S
   INCI: Hexyldecyl Stearate
   Hersteller: Cognis Deutschland GmbH (Henkel)
19. Finsolv® TN
   INCI: C₁₂-C₁₅ Alkyl Benzoate
   Hersteller. Finetex (Nordmann, Rassmann)
20. Fitoderm®
   INCI: Squalane
   Hersteller. Hispano-Quimica-S.A., Nordmann-Rassmann
21. Indinyl® CA
   INCl: Water (and) Cassia angustifolia sed polysaccharide
   Hersteller: Laboratoire Serobiologique
22. Lactolan® LS 5879
   INCI: Hydrolyzed milk protein
   Hersteller: Laboratoire Serobiologique
23. Locron® L
   INCI: Aluminum Chlorohydrate (Lösung: 50 Gew.-% in Wasser)
   Hersteller: Clariant
24. Melhydran®
   INCI: Honey Extract
   Hersteller: Laboratoires Serobiologiques (Cognis)
25. Myritol® 331
   INCI: Cocoglycerides
   Hersteller: Cognis Deutschland GmbH (Henkel)
26. Neo Heliopan® 303
   INCI: Octocrylene
   Hersteller: Haarmann & Reimer
27. Neo Heliopan® BB
   INCI: Benzophenone 3
   Hersteller: Haarmann & Reimer
28. Neo Heliopan® MBC
   INCl: 4-Methylbenzylidene Camphor
   Hersteller: Haarmann & Reimer
29. Neo Heliopan® OS
   INCl: Ethylhexyl Salicylate
   Hersteller: Haarmann & Reimer
30. Phytalbumin® HGP
   INCI: Glycine Soja (Soybean) Protein
   Hersteller. Laboratoire Serobiologique
31. Rezal® 36GC
   INCI: Aluminium-Zirconium Tetrachlorohydrex Gly (Lösung)
   Hersteller. Reheis
32. Rezal® 67
   INCI: Aluminium Zirconium Pentachlorohydrat
   Hersteller: Reheis
33. Urvinol® T 150
   INCI: Octyl Triazone
   Hersteller: BASF

## Patentansprüche

1. Emulgator-Gemisch **dadurch gekennzeichnet, daß**
(a) 0,1- 60 Gew.-% eines Fettalkohols oder Fettalkohol-Gemisches
(b) 30 - 97 Gew.-% eines ethoxylierten Fettalkohols oder Gemisches ethoxylierter Fettalkohole
(c) 0,1- 20 Gew.-% eines Dialk(en)ylethers, eines Dialk(en)ylcarbonats oder eines beliebigen Gemisches dieser Komponenten
(d) weniger als 10 Gew.-% Wasser.
enthalten sind.

2. Zusammensetzung gemäß Anspruch 1, **dadurch gekennzeichnet, daß** der Fettalkohol ausgewählt ist aus der Gruppe der C₁₂ - C₂₄-Fettalkohole.

3. Zusammensetzung gemäß einem der Ansprüche 1 bis 2, **dadurch gekennzeichnet, daß** der ethoxylierte Fettalkohol (b) ein Gemisch aus
(b1) C₁₂- C₂₄-Fettalkoholen mit 1 - 5 EO
(b2) C₁₂-C₂₄-Fettalkoholen mit 6 - 35 EO
enthält.

4. Zusammensetzung gemäß einem der Ansprüche 1 bis 3, **dadurch gekennzeichnet, daß** der ethoxylierte Fettalkohol (b) ein Gemisch aus
(b1) C₁₂ - C₂₄-Fettalkoholen mit 1 - 5 EO,
(b2) C₁₂ - C₂₄-Fettalkoholen mit 6 - 16 EO und
(b3) C₁₂ - C₂₄-Fettalkoholen mit 18 - 35 EO
enthält.

5. Zusammensetzung gemäß einem der Ansprüche 1 bis 4, **dadurch gekennzeichnet, daß** der Dialk(en)ylether ausgewählt ist aus der Gruppe der C₆ - C₃₂-Dialk(en)ylether.

6. Zusammensetzung gemäß einem der Ansprüche 1 bis 5, **dadurch gekennzeichnet, daß** das Dialk(en)ylcarbonat ausgewählt ist aus der Gruppe der C₆ - C₃₂-Dialk(en)ylcarbonate.

7. Zusammensetzung gemäß einem der Ansprüche 1 bis 6, **dadurch gekennzeichnet, daß** zusätzlich ein Partialglycerid enthalten ist.

8. Zusammensetzung gemäß Anspruch 7, **dadurch gekennzeichnet, daß** das Partialglycerid ausgewählt ist aus der Gruppe der C₁₂ - C₁₈-Partialglyceride.

9. Zusammensetzung gemäß einem der Ansprüche 1 bis 8, **dadurch gekennzeichnet, daß**
(a) 0,1- 60 Gew.-% eines C₁₂ - C₂₄-Fettalkohols,
(b1) 10 - 65 Gew.-% eines C₁₂ - C₂₄-Fettalkohols mit 1 - 5 EO,
(b2) 10 - 30 Gew.-% eines C₁₂ - C₂₄-Fettalkohols mit 6 - 16 EO,
(b3) 10 - 20 Gew.-% eines C₁₂ - C₂₄-Fettalkohols mit 18 - 35 EO, und
(c) 0,1 bis 15 Gew.-% eines C₆ - C₃₂-Dialk(en)ylethers, eines C₆ - C₃₂₋Dialk(en)ylcarbonats oder eines beliebigen Gemisches dieser Komponenten
enthalten ist.

10. Zusammensetzung gemäß einem der Ansprüche 1 bis 9, **dadurch gekennzeichnet, daß**
(a) 0,1 - 60 Gew.-% eines C₁₂ - C₂₄-Fettalkohols,
(b1) 10 - 65 Gew.-% eines C₁₂ - C₂₄-Fettalkohols mit 1 - 5 EO,
(b2) 10 - 30 Gew.-% eines C₁₂ - C₂₄-Fettalkohols mit 6 -16 EO,
(b3) 10 - 20 Gew.-% eines C₁₂ - C₂₄-Fettalkohols mit 18 - 35 EO,
(c) 0,1 bis 15 Gew.-% eines C₆ - C₃₂-Dialk(en)ylethers, eines C₆ - C₃₂₋Dialk(en)ylcarbonats oder eines beliebigen Gemisches dieser Komponenten
(e) 0,1 bis 10 Gew.% eines C₁₂ - C₁₈-Partialglycerids
enthalten ist.

11. Verwendung der Emulgator-Gemische gemäß einem der Ansprüche 1 bis 10 zur Herstellung kosmetischer und pharmazeutischer Mittel.

12. Kosmetische Zubereitung enthaltend 2 -20 Gew.-% der Emulgator-Gemische gemäß einem der Ansprüche 1 bis 10 bezogen auf die Gesamtzusammensetzung, **dadurch gekennzeichnet, daß** sie Antitranspirant-Wirkstoffe enthält.

13. Kosmetische Zubereitung enthaltend 2-20 Gew.% der Emulgator-Gemische gemäß einem der Ansprüche 1-10 bezogen auf die Gesamtzusammensetzung, **dadurch gekennzeichnet, daß** sie einen UV-Lichtschutzfilter enthält.

14. Kosmetische Zubereitung gemäß Anspruch 12, **dadurch gekennzeichnet, daß** sie eine Viskosität von 1000 - 20000 mPa·s bei 23 °C aufweist.

15. Kosmetische Zubereitung gemäß Anspruch 12, **dadurch gekennzeichnet, daß** sie eine Viskosität von wenigsten 20 - 800 Pa.s bei 23° C aufweist.

16. Verwendung der Emulgator-Gemische gemäß einem der Ansprüche 1 bis 10 zur Stabilisierung von dünnflüssigen O/W Emulsionen mit Viskositäten von 100 - 20000 mPa·s (Brookfield, RVF, Spindel 5,10 Upm, 23°C).

## Claims

1. An emulsifier mixture, **characterized in that** it contains
(a) 0.1 to 60% by weight of a fatty alcohol or fatty alcohol mixture,
(b) 30 to 97% by weight of an ethoxylated fatty alcohol or mixture of ethoxylated fatty alcohols,
(c) 0.1 to 20% by weight of a dialk(en)yl ether, a dialk(en)yl carbonate or a mixture of these components,
(d) less than 10% by weight of water.

2. A composition as claimed in claim 1, **characterized in that** the fatty alcohol is selected from the group of C₁₂₋₂₄ fatty alcohols.

3. A composition as claimed in any of claims 1 to 2, **characterized in that** the ethoxylated fatty alcohol (b) is a mixture of
(b1) C₁₂₋₂₄ fatty alcohols + 1 - 5 EO,
(b2) C₁₂₋₂₄ fatty alcohols + 6 - 35 EO.

4. A composition as claimed in any of claims 1 to 3, **characterized in that** the ethoxylated fatty alcohol (b) is a mixture of
(b1) C₁₂₋₂₄ fatty alcohols + 1 - 5 EO,
(b2) C₁₂₋₂₄ fatty alcohols + 6 - 16 EO and
(b3) C₁₂₋₂₄ fatty alcohols + 18 - 35 EO.

5. A composition as claimed in any of claims 1 to 4, **characterized in that** the dialk(en)yl ether is selected from the group of C₆₋₃₂ dialk(en)yl ethers.

6. A composition as claimed in any of claims 1 to 5, **characterized in that** the dialk(en)yl carbonate is selected from the group of C₆₋₃₂ dialk(en)yl carbonates.

7. A composition as claimed in any of claims 1 to 6, **characterized in that** a partial glyceride is additionally present.

8. A composition as claimed in any of claims 1 to 7, **characterized in that** the partial glyceride is selected from the group of C₁₂₋₁₈ partial glycerides.

9. A composition as claimed in any of claims 1 to 8, **characterized in that** it contains
(a) 0.1 - 60% by weight of a C₁₂₋₂₄ fatty alcohol,
(b1) 10 - 65% by weight of a C₁₂₋₂₄ fatty alcohol + 1 - 5 EO,
(b2) 10 - 30% by weight of a C₁₂₋₂₄ fatty alcohol + 6 -16 EO,
(b3) 10 - 20% by weight of a C₁₂₋₂₄ fatty alcohol + 18 - 35 EO and
(c) 0.1 to 15% by weight of a C₆₋₃₂ dialk(en)yl ether, a C₆₋₃₂ dialk(en)yl carbonate or a mixture of these components.

10. A composition as claimed in any of claims 1 to 9, **characterized in that** it contains
(a) 0.1 - 60% by weight of a C₁₂₋₂₄ fatty alcohol,
(b1) 10 - 65% by weight of a C₁₂₋₂₄ fatty alcohol + 1 - 5 EO,
(b2) 10 - 30% by weight of a C₁₂₋₂₄ fatty alcohol + 6 - 16 EO,
(b3) 10 - 20% by weight of a C₁₂₋₂₄ fatty alcohol + 18 - 35 EO,
(c) 0.1 to 15% by weight of 6₆₋₃₂ dialk(en)yl ether, a C₆₋₃₂ dialk(en)yl carbonate or a mixture of these components,
(e) 0.1 to 10% by weight of a C₁₂₋₂₈ partial glyceride.

11. The use of the emulsion mixtures claimed in any of claims 1 to 10 for the production of cosmetic and pharmaceutical preparations.

12. A cosmetic preparation containing 2 to 20% by weight of the emulsifier mixtures claimed in any of claims 1 to 10, based on the overall composition, **characterized in that** it contains antiperspirant components.

13. A cosmetic preparation containing 2 to 20% by weight of the emulsifier mixtures claimed in any of claims 1 to 10, based on the overall composition, **characterized in that** it contains a UV protection filter.

14. A cosmetic preparation as claimed in claim 12, **characterized in that** it has a viscosity of 1,000 to 20,000 mPa.s at 23°C.

15. A cosmetic preparation as claimed in claim 12, **characterized in that** it has a viscosity of at least 20 to 800 Pa.s at 23°C.

16. The use of the emulsifier mixtures claimed in any of claims 1 to 10 for stabilizing thinly liquid o/w emulsions with viscosities of 100 to 20,000 mPa.s (Brookfietd, RVF, spindle 5, 10 r.p.m., 23°C).

## Revendications

1. Mélange d'émulsionnants,
**caractérisé en ce qu'**
il contient :
a) 0,1 - 60 % en poids d'un alcool gras ou d'un mélange d'alcools gras,
b) 30 - 97 % en poids d'un alcool gras éthoxylé ou d'un mélange d'alcools gras éthoxylés,
c) 0,1 - 20 % en poids d'un éther de dialc(èn)yle, d'un carbonate de dialc(èn)yle ou d'un mélange quelconque de ces composants,
d) moins de 10 % en poids d'eau.

2. Composition selon la revendication 1,
**caractérisée en ce que**
l'alcool gras est choisi dans le groupe des alcools gras en C₁₂- C₂₄.

3. Composition selon l'une des revendications 1 et 2,
**caractérisée en ce que**
l'alcool gras éthoxylé b) contient un mélange constitué de :
(b1) d'alcools gras en C₁₂ - C₂₄ avec 1 - 5 OE
(b2) d'alcools gras en C₁₂- C₂₄ avec 6 - 35 OE.

4. Composition selon l'une des revendications 1 à 3,
**caractérisée en ce que**
l'alcool gras éthoxylé (b) contient un mélange constitué :
(b1) d'alcools gras en C₁₂ - C₂₄ avec 1 - 5 OE
(b2) d'alcools gras en C₁₂ - C₂₄ avec 6 - 16 OE et
(b3) d'alcools gras en C₁₂ - C₂₄ avec 18 - 35 OE

5. Composition selon l'une des revendications 1 à 4,
**caractérisée en ce que**
l'éther de dialc(èn)yle est choisi dans le groupe des éthers de dialc(èn)yle en C₆ -C₃₂.

6. Composition selon l'une des revendications 1 à 5,
**caractérisée en ce que**
le carbonate de dialc(èn)yle est choisi dans le groupe des carbonates de dialc(èn)yle en C₆-C₃₂.

7. Composition selon l'une des revendications 1 à 6,
**caractérisée en ce qu'**
elle contient en plus un glycéride partiel.

8. Composition selon la revendication 7,
**caractérisée en ce que**
le glycéride partiel est choisi dans le groupe des glycérides partiels en C₁₂-C₁₈.

9. Composition selon l'une des revendications 1 à 8,
**caractérisée en ce qu'**
elle contient :
a) 0,1 - 60 % en poids d'un alcool gras en C₁₂ - C₂₄,
(b1) 10 - 65 % en poids d'un alcool gras en C₁₂- C₂₄ avec 1 - 5 OE,
(b2) 10 - 30 % en poids d'un alcool gras en C₁₂ - C₂₄ avec 6 - 16 OE,
(b3) 10 - 20 % en poids d'un alcool gras en C₁₂- C₂₄ avec 18 - 35 OE et
c) 0,1 à 15 % en poids d'un éther de dialc(èn)yle en C₆ - C₃₂, d'un carbonate de dialc(èn)yle en C₆ -C₃₂ ou d'un mélange quelconque de ces composants.

10. Composition selon l'une des revendications 1 à 9,
**caractérisée en ce qu'**
elle contient :
a) 0,1 - 60 % en poids d'un alcool gras en C₁₂ - C₂₄ ,
(b1) 10 - 65 % en poids d'un alcool gras en C₁₂- C₂₄ avec 1 - 5 OE,
(b2) 10 - 30 % en poids d'un alcool gras en C₁₂- C₂₄ avec 6 - 16 OE,
(b3) 10 - 20 % en poids d'un alcool gras en C₁₂- C₂₄ avec 18 - 35 OE,
c) 0,1 à 15 % en poids d'un éther de dialc(èn)yle en C6 - C32, d'un carbonate de dialc(èn)yle en C₆ - C₃₂ ou d'un mélange quelconque de ces composants,
e) 0,1 à 10 % en poids d'un glycéride partiel en C₁₂ - C₁₈.

11. Utilisation des mélanges d'émulsionnants selon l'une des revendications 1 à 10, pour la préparation de produits cosmétiques et pharmaceutiques.

12. Préparation cosmétique contenant 2 - 20 % en poids des mélanges d'émulsionnants selon l'une des revendications 1 à 10, rapporté à la composition totale,
**caractérisée en ce qu'**
elle contient des substances actives anti-transpirantes.

13. Préparation cosmétique contenant 2 - 20 % en poids des mélanges d'émulsionnants selon l'une des revendications 1 à 10, rapporté à la composition totale,
**caractérisée en ce qu'**
elle contient un filtre de protection contre les UV.

14. Préparation cosmétique selon la revendication 12,
**caractérisée en ce qu'**
elle présente une viscosité de 1000 - 20000 mPa.s à 23°C.

15. Préparation cosmétique selon la revendication 12,
**caractérisée en ce qu'**
elle présente une viscosité d'au moins 20 - 800 Pa.s à 23 °C.

16. Utilisation des mélanges d'émulsionnants selon l'une des revendications 1 à 10 pour la stabilisation d'émulsions huile-dans-l'eau, fluides, présentant des viscosités de 100 - 20 000 mPa.s (Brookfield, RVF, arbre 5,10 t/min, 23°C).
